# EUROPEAN PATENT SPECIFICATION

(11) **EP 0 744 165 B1**
(45) Date of publication and mention of the grant of the patent: **19.02.2003**
(21) Application number: 96303742.9
(22) Date of filing: 24.05.1996
(51) Int. Cl.: A61F 2/08

(54) **Device for the repair of the rotator cuff of the shoulder**
Vorrichtung zum Wiederherstellen der Schulterdreherklappe
Dispositif de réparation de la coiffe des rotateurs de l'épaule

(30) Priority: 25.05.1995 GB 9510637
(43) Date of publication of application: 27.11.1996
(73) Proprietor: ELLIS DEVELOPMENTS LIMITED, Nottingham NG3 2AP (GB)
(72) Inventor: Ellis, Julian Garth, Fiskerton,Southwell,Notts, NG25 OUJ (GB); Neumann, Lars, Mapperley Park, Nottingham, NG3 5EH (GB)
(74) Representative: Goodwin, Mark

(56) References cited:
- WO-A-90/12551
- WO-A-91/03993
- WO-A-92/10218
- FR-A- 2 638 349
- FR-A- 2 690 073
- FR-A- 2 710 829

## Description

The present invention relates to a device for the repair of the rotator cuff of the shoulder.

The rotator cuff is the assembly of tendons and ligaments around the shoulder joint that holds the ball of the humeral head onto the socket of the glenoid and is involved in the control of movement of the shoulder. There are four tendons that form the cuff - the supraspinatus, infraspinatus, teres minor and subscapularis tendons. From about the age of 35 years onwards there is an increasing risk of this cuff becoming torn - often as a result of injury - causing pain and disability. This condition of a torn cuff becomes progressively more common with age until at the age of 70 years approximately 30% of people probably have rotator cuff tears.

Non-surgical management is usually successful for small tears in about 30% of cases. However, 70% do not respond to conservative treatment. When attempts are made to repair the cuff surgically common reasons for failure are firstly re-rupture of the tear for mechanical reasons - the force transmitted through the cuff on raising the arm can amount to body weight - and secondly because of stretching of the soft tissues of the cuff after repair results in loss of the stabilising effect of the cuff.

Frequently for the repair of the rotator cuff, a multiplicity of suture threads is used. However, sometimes this method of reinforcement is insufficient because the sutures cut through the bone or tendon. To deal with that a reinforcing textile fabric is required. The use of a hood as described in PCT International Patent Appln.No.WO 91/03993 is one example. This discloses a woven or knitted flexible fabric, the openness of the mesh being chosen as to allow tissue ingrowth without being so open that the structural integrity is lost. The use of a hood does, however, usually require the removal of the head of the humerus, which, when the quality of the bone is good and the articular surfaces of the joint satisfactory, may not be desirable.

The object of the present invention is to provide a device for repair of the rotator cuff which provides a patch to reinforce the rotator cuff and does not require the removal of the head of the humerus.

Document FR-A-2 638 349 discloses a device for repair of the rotator cuff according to the preamble of claim 1 which does not require the removal of the humeral head.

According to the present invention there is provided a device for use in the repair of the rotator cuff of a shoulder, said shoulder comprising a humeral head and rotator cuff, the device having the features of claim 1.

The flexible member is preferably strengthened or reinforced at least at the site or sites where it is to be attached to the humeral head and at the site or sites where it is to be secured to the rotator cuff. The flexible member is preferably secured to the humeral head by a rigid mechanical fastening such as a screw of the like which extends through the flexible member and into the humeral head. The flexible member is preferably fastened to the rotator cuff by flexible fastenings such as sutures. The flexible fastenings may secure the rotator cuff to one or more sites at or adjacent the edge of the flexible member remote from the site where the flexible member is secured to the humeral head. In addition further flexible fastenings may secure the rotator cuff to the flexible member at one or more sites intermediate the connection of the flexible member to the humeral head and the first mentioned flexible fastening of the flexible member to the rotator cuff. Preferably the further flexible fastenings are transosseal sutures which may extend from the flexible member to the rotator cuffs through one or more tunnels formed in the humeral head.

The main load bearing portion of the device is provided by the reinforcing cords preferably of braided polyester which are held onto the flexible member for example by stitching. The stitching can be effected by conventional stitching methods, for example by a conventional sewing machine, or by stitching using an embroidery machine which is computer controlled so as-to achieve repeatability. The mesh is preferred so that sutures can be applied from anywhere on the rotator cuff to attach to the flexible member. The surgeon usually will not know before a repair operation where the cuff is torn or the size of the tear, so a repair device whereby any undamaged part of the cuff can be secured to the flexible member is advantageous. This connection transfers the load from the rotator cuff to the connection and the connection transfers the load to the flexible member. The close proximity of the flexible member to the cuff and the subsequent growth of the tissue through the holes in the flexible member will enable full incorporation of the flexible member within the cuff after a period of new tissue growth.

The aim of the invention is to repair the rotator cuff particularly when the humeral bone has a degenerated tendon edge and there is a weak thin bone at the back of the humeral bone and reinstate the cuff to its previous strength. However, degenerated tissues need reinforcement during healing. Ingrowth of fibrous tissue will take place if the structure permits. The preferred embodiment of the invention will induce strengthening of the tissue area after healing has taken place.

In a preferred embodiment of the invention the flexible member comprises a mesh or like textile material with perforations which may be of uniform or non-uniform size and regularly or irregularly arranged. The mesh may be made by a number of methods, including sewing, weaving and by embroidery. The mesh is preferably formed so that the holes are spaced approximately 2 mm apart and are approximately 1 mm square. If woven, the flexible member is preferably a mock leno or leno woven, in which the weft threads are locked with respect to the warp threads. In one preferred embodiment a mock leno weave comprises a warp of 550 decitex polyester yarn and a weft of twisted polyester and the holes are about 0.1 x 0.5 cm in size there being about 20 holes per cm².

The strength of the mesh is preferably such that it should carry a minimum load of approximately the body weight of the patient, preferably no less than 3000 N. The use of embroidery to form the mesh is particularly preferred since it has the advantage that the mesh can be locally reinforced using a different type of thread or a different density of mesh. In a preferred embodiment the main mesh is made using polyester fibre bundles 0.35 mm in diameter. The reinforcement is also preferably of polyester, but of larger diameter, for example from 0.5 to 1.5 mm diameter preferably formed by braiding. However, the mesh may be formed by weaving, knitting, lace, embroidery or other fibre assembly process, alone or in combination. The shape of the mesh is preferably preformed for example in the approximate shape of a triangle to simplify installation and to provide reinforcement in the optimum position, preferably at one corner, to provide maximum reinforcement of the lesion. The mesh will assist in the carrying of load across to the strongest parts of the device, the cords, although some of the load will be carried by the mesh itself. The cords also provide a convenient method of preventing the mesh unravelling at the edge.

The whole device helps to carry load to the screw (or other fixation device) which attaches the cuff via the flexible member to the humerus.

A specific embodiment of the invention will now be described by way of example with reference to the accompanying drawings in which:-
- Fig.1: illustrates a mesh; and
- Fig.2: shows how a mesh is used to repair a rotator cuff.

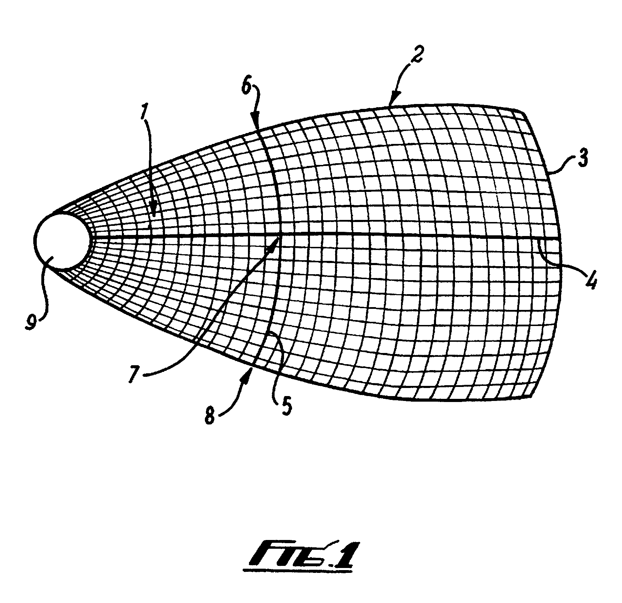

The preferred shape of this embodiment of the invention is shown in Fig.1 and is a mesh of substantially triangular overall shape. The mesh is reinforced along the edges 2 and 3 in a line 4 from the apex to the base of the triangular mesh and in a line 5 across the mesh at approximately a half way position. This line 5 is intended to provide strengthened attachment locations for sutures at points 6, 7 and 8. There is also provided reinforcement around a hole 9 formed at the apex of the triangle which is intended to receive rigid fastening means such as a screw. Instead of a screw, a toggle may be used, a biodegradable screw might be used or an anchor device, such as a Mitek bone anchor.

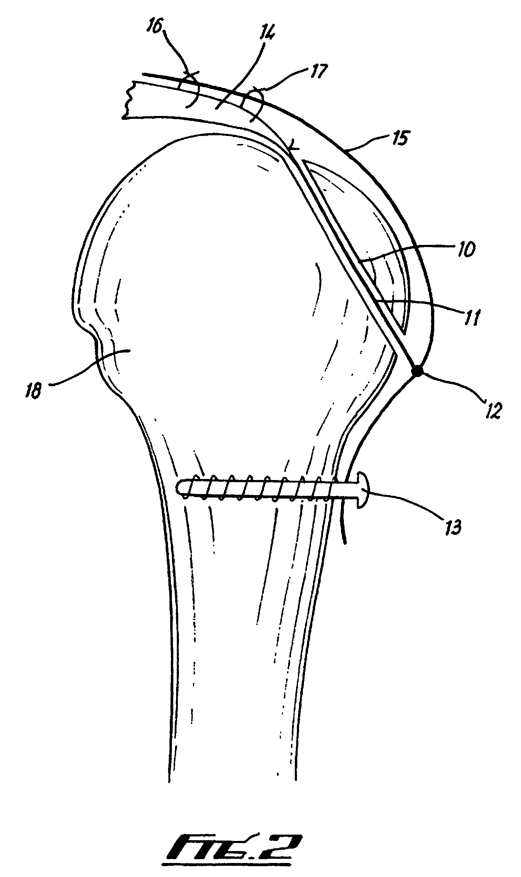

As shown in Fig.2, in order to fit the mesh device of the invention to a patient, a plurality of tunnels, one of which referenced 10 is shown, are drilled through the humeral head. Flexible fastening means such as transosseal sutures 11 extend through the tunnels 10. The sutures are fastened at one end to the rotator cuff tendon 14 and at the other end to the mesh 15 at attachment points 6, 7 and 8 (Fig.1). A screw 22 is inserted through the hole 9 in the prosthesis 15 and screwed into the humeral head. This is the point at which load is concentrated so that the load is taken up through the screw to the humeral neck. Flexible fastening means such as sutures 16 and 17 join the edge of the mesh to the rotator cuff 14.

Many different types of fibre may be used to form the invention, including silk, metallic wire, polyester, carbon, nylon, polypropylene, polyethylene, PTFE. The fibres may be absorbable fibres including polglycolic acid and polyglactin, alone or in combination.

The device of the invention may be impregnated with one or more growth factors or angiogenic or neurogenic materials that may stimulate the production of blood vessels, nerves or other types of tissue.

## Claims

1. A device (15) for use in the repair of the rotator cuff of a shoulder said shoulder comprising a humeral head (18), and said device comprising a flexible member (15), said member having means (13) for fixing it to the humeral head and means (11,16,17) adapted to secure the rotator cuff (14) to the member **characterised in that** the flexible member comprises one or more additional reinforcement fibres (4,5) to provide reinforcement.

2. A device as claimed in Claim 1, wherein the flexible member comprises a mesh.

3. A device as claimed in Claim 1 or Claim 2, wherein the flexible member is of substantially triangular shape.

4. A device as claimed in any preceding claim, wherein the flexible member is reinforced across at least a part thereof (2, 3, 4).

5. A device as claimed in any one of Claims 1 to 4, wherein the fixing means comprises a flexible fastening means (16, 17).

6. A device as claimed in any one of Claims 1 to 4, wherein the fixing means comprises a rigid fixing means (13).

7. A device as claimed in any preceding claim, wherein the flexible member can support a load of at least 3000 N.

8. A device as claimed in any preceding claim, wherein the flexible member is formed by weaving, knitting, embroidery, other fibre assembly process or combination thereof.

9. A device as claimed in any preceding claim impregnated with one or more growth factors, angiogenic or neurogenic materials.

## Patentansprüche

1. Vorrichtung (15) zur Wiederherstellung der Rotatorenmanschette einer Schulter, wobei die Schulter einen Humeruskopf (18) umfaßt, und wobei die Vorrichtung ein flexibles Glied (15) umfaßt, das eine Vorrichtung (13) zur Befestigung am Humeruskopf sowie Vorrichtungen (11, 16, 17) aufweist, die geeignet sind, die Rotatorenmanschette (14) an dem Glied zu befestigen,
**dadurch gekennzeichnet, daß** das flexible Glied einen oder mehrere zusätzliche Verstärkungsfäden (4, 5) umfaßt, um eine Verstärkung zu erzielen.

2. Vorrichtung nach Anspruch 1,
bei der das flexible Glied ein Netz umfaßt.

3. Vorrichtung nach Anspruch 1 oder Anspruch 2,
bei der das flexible Glied von im wesentlichen dreieckiger Form ist.

4. Vorrichtung nach einem vorhergehenden Anspruch,
bei der das flexible Glied zumindest über einen Teil (2, 3, 4) verstärkt ist.

5. Vorrichtung nach einem der Ansprüche 1 bis 4,
bei der die Befestigungsvorrichtung eine flexible Befestigungsvorrichtung (16, 17) umfaßt.

6. Vorrichtung nach einem der Ansprüche 1 bis 4,
bei der die Befestigungsvorrichtung eine starre Befestigungsvorrichtung (13) umfaßt.

7. Vorrichtung nach einem vorhergehenden Anspruch,
bei der das flexible Glied mit einer Last von mindestens 3000 N beaufschlagbar ist.

8. Vorrichtung nach einem vorhergehenden Anspruch,
bei der das flexible Glied durch Weben, Stricken, Sticken, ein sonstiges Faseranordnungsverfahren oder eine Kombination dieser Verfahren gebildet wird.

9. Vorrichtung nach einem vorhergehenden Anspruch, imprägniert mit einem oder mehreren Wachstumsfaktoren, gefäßbildenden oder neurogenen Stoffen.

## Revendications

1. Dispositif (15) destiné à être utilisé pour la réparation de la coiffe des rotateurs d'une épaule, ladite épaule comprenant une tête humérale (18), et ledit dispositif comprenant un organe flexible (15), ledit organe possédant des moyens (13) destinés à fixer ledit organe à la tête humérale et des moyens (11, 16, 17) adaptés pour fixer la coiffe (14) des rotateurs audit organe, **caractérisé en ce que** l'organe flexible comprend une ou plusieurs fibre(s) de renfort additionnelle(s) (4, 5) pour procurer un renforcement.

2. Dispositif suivant la revendication 1, dans lequel l'organe flexible comprend un maillage.

3. Dispositif suivant la revendication 1 ou 2, dans lequel l'organe flexible et de forme sensiblement.riangulaire.

4. Dispositif suivant l'une quelconque des revendications précédentes, dans lequel l'organe flexible est renforcé transversalement sur au moins une partie de ce dernier (2, 3, 4).

5. Dispositif suivant l'une quelconque des revendications 1 à 4, dans lequel les moyens de fixation comprennent des moyens de fixation flexibles (16, 17).

6. Dispositif suivant l'une quelconque des revendications 1 à 4, dans lequel les moyens de fixation comprennent des moyens de fixation rigides (13).

7. Dispositif suivant l'une quelconque des revendications précédentes, dans lequel l'organe flexible peut supporter une charge d'au moins 3000 N.

8. Dispositif suivant l'une quelconque des revendications précédentes, dans lequel l'organe flexible est formé par tissage, tricotage, brodage ou autres procédés d'assemblage de fibres, ou une combinaison de ceux-ci.

9. Dispositif suivant l'une quelconque des revendications précédentes, imprégné par un ou plusieurs facteur(s) de croissance, des matières angiogéniques ou neurogènes.
